Europäisches Patentamt

European Patent Office

Office européen des brevets

(11) Veröffentlichungsnummer : **0 130 434**
**B1**

(12) **EUROPÄISCHE PATENTSCHRIFT**

(45) Veröffentlichungstag der Patentschrift :
27.05.87

(21) Anmeldenummer : 84106844.8

(22) Anmeldetag : 15.06.84

(51) Int. Cl.⁴ : **G 01 N 33/577, G 01 N 33/80**

(54) **Diagnostisches Testsystem.**

(30) Priorität : 01.07.83 DE 3323645

(43) Veröffentlichungstag der Anmeldung :
09.01.85 Patentblatt 85/02

(45) Bekanntmachung des Hinweises auf die Patenterteilung : 27.05.87 Patentblatt 87/22

(84) Benannte Vertragsstaaten :
AT BE CH DE FR GB IT LI LU NL SE

(56) Entgegenhaltungen :
EP-A- 0 051 748
DE-A- 3 145 007

(73) Patentinhaber : Biotest-Serum-Institut GmbH
Flughafenstrasse 4
D-6000 Frankfurt-Niederrad (DE)

(72) Erfinder : Uthemann, Horst, Dr. Dipl.-Chem.
Sachsenh. Landwehrweg 66
D-6000 Frankfurt 70 (DE)

(74) Vertreter : Wolff, Hans Joachim, Dr.jur. Dipl.-Chem. et
al
Beil, Wolff & Beil Rechtsanwälte Postfach 80 01 40
Adelonstrasse 58
D-6230 Frankfurt am Main 80 (DE)

**Beschreibung**

Die Erfindung betrifft ein diagnostisches Testsystem bestehend aus einer festen Phase aus einem proteinbindungsfähigen Polystyrol oder einer Polyesterfolie, die mindestens auf einer Seite mit einer gleichmäßigen gegenüber bestimmten Reagentien haftfähigen Pigmentlackmattierungsschicht versehen ist, auf die in räumlich voneinander getrennten Kompartimenten oder Feldern Reagentien aufgetrocknet sind.

Unter diagnostischem Testsystem versteht man beispielsweise Blutgruppenidentitätskarten, die insbesondere ihre Anwendung im Bed side-Test finden oder Mikrotiterplatten zur Blutgruppendiagnostik, die ihre Anwendung in der Blutgruppenserologie, in Blutbanken, Krankenhäusern und bei Vaterschaftsbestimmungen finden.

Aus dem DE-GM-80 29 596 ist eine Blutgruppenidentitätskarte aus einer mit Pigmentlackmattierungsschicht versehenen Polyesterfolie bekannt, die auf ihrer Oberfläche auf räumlich voneinander getrennten Feldern festhaftende trockne Schichten aus im wesentlichen reinen Antiseren aufweist.

Gegenüber früheren Blutgruppenidentitätskarten mit angetrockneten Antiseren, die als Haftvermittler Dextran verwendeten, wobei das Dextran-Antiserum-Gemisch nicht direkt mit dem zu testenden Blut vermischt werden konnte, sondern erst mit Wasser aufgelöst werden mußte, hat die in der DE-GM-80 29 596 beschriebene Karte den Vorteil, daß auf der mit Pigmentlackmattierung beschichteten Polyesterfolie, wie sie als Zeichenfolie auf dem Markt ist, Antiseren ohne jegliche haftvermittelnden Zusätze ausgezeichnet haften. Dadurch entfällt ein Haftmittel, wie es bei früheren Karten erforderlich war, und zugegebenes Blut löst beim Verrühren die angetrockneten Antiseren auf, so daß auf das Auflösen mit Wasser verzichtet werden kann und der Test sehr leicht und schnell durchführbar ist.

Aus dem DE-GM-81 37 962 ist eine Mikrotiterplatte zur Blutgruppendiagnostik bekannt, die mit einer Vielzahl von Näpfchen aus einem proteinbindungsfähigen Kunststoff ausgestattet ist und deren Näpfchenböden festhaftende trockne Schichten aus im wesentlichen reinen Antiseren aufweisen.

Gegenüber früheren Mikrotiterplatten, in die flüssige Antiseren entweder unmittelbar vor der Untersuchung in die Näpfchen eingefüllt oder auf Vorrat eingefüllt und eingefroren wurden, hat die in der DE-GM-81 37 962 beschriebene Mikrotiterplatte den Vorteil, daß auf dem starren, transparenten, proteinbindungsfähigen Kunststofftrockne Antiseren ohne jegliche haftvermittelnden Zusätze ausgezeichnet haften, Antiseren, die in die Näpfchen eingebracht werden, eine festhaftende Schicht auf den Böden der Näpfchen bilden und beim Transport kein Ablösen der Schicht erfolgt.

Das Einfüllen flüssiger Antiseren entfällt und vor der Anwendung erübrigt sich somit ein Rehydratisieren. Es wird lediglich ein Tropfen Kochsalzaufschwemmung der zu untersuchenden Blute zugegeben, wobei sich durch leichtes Schütteln die Antiseren lösen.

Nach kurzem Zentrifugieren (z. B. 2 Minuten bei 1 000 U/Min.) — ohne vorausgehende Inkubation — wird aufgeschüttelt und ausgewertet.

Neben all den Vorteilen, die diese vorstehend beschriebenen diagnostischen Testsysteme gegenüber früheren Systemen aufweisen, besitzen sie jedoch den Nachteil, daß ihre Lagerfähigkeit bei höheren Temperaturen oder bei der sogenannten Wechsellagerung, d. h. Wechsel zwischen höheren und niedrigeren Temperaturen begrenzt ist.

Die auf den festen Phasen angetrockneten, im wesentlichen reinen Antiseren werden z. B. aus Citratplasmen durch Rekalzifizierung gewonnen. Der Rekalzifizierungsvorgang ist ein Gleichgewichtsprozeß, der nicht zur völligen Ausfällung aller am Gerinnungsvorgang beteiligter Proteine führt.

Um Denaturierung der Proteine zu verhindern, muß eine gewisse Restfeuchte im Produkt vorhanden sein. Bei Lagerung bei höheren Temperaturen finden in Gegenwart der Restfeuchte Wechselwirkungen der Proteine untereinander statt, die zu Gelbildung führen. Die Vielfalt der Proteine und ihre Wechselwirkungen untereinander machen es unmöglich, genaue Angaben über schwerlösliche oder zur Gelbildung führende Proteine zu machen. Als störende Faktoren kommen vor allem Denaturierungs- und Abbauprodukte der am Gerinnungsablauf beteiligten Proteine in Betracht. Der Faktor XIII, ein fibrinstabilisierender Gerinnungsfaktor, kann zu Quervernetzungen führen, und es ist mit Fibronectin, dem CIG (cold insoluble globulin), einem Fibrinabbauprodukt, zu rechnen, das bei Zimmertemperatur nicht in Lösung geht.

Bei Zugabe von Blut oder einer Erythrozytensuspension zu den angetrockneten Antiseren kann eine solche Gelbildung ein vollständiges Auflösen verhindern, was zu falsch positiven Resultaten führt, da die Gelbildung wie eine Agglutination aussieht.

Der Erfindung liegt die Aufgabe zugrunde, die Lagerfähigkeit diagnostischer Testysteme, bei denen auf eine feste Phase Reagentien, wie Antiseren, aufgetrocknet sind, zu verbessern.

Diese Aufgabe wird erfindungsgemäß dadurch gelöst, daß in einem gattungsgemäßen diagnostischen Testsystem als aufgetrocknete Reagentien anstelle herkömmlicher Antiseren oder deren Immunglobulin-Fraktionen, monoklonale Antikörper mit Spezifität gegen bestimmte Antigene, insbesondere Blutgruppenantigene, verwendet werden.

Man fand, daß aufgrund der Reinheit der monoklonalen Antikörper — sie sind praktisch frei von Fremdproteinen — keine Nachgerinnung oder störende Gelbildung bei Lagerung bei höheren Temperaturen oder Raumtemperatur stattfindet.

Gegen die Verwendung von monoklonalen Antikörpern für den erfindungsgemäßen Zweck bestand jedoch ein Vorurteil. Es war zu erwarten, daß aufgrund der hohen Verdünnung, in der monoklonale Antikörper vorliegen, und der Abwesenheit von Schutzkolloiden, wie z. B. Albumin, keine lange Haltbarkeit besonders bei Raumtemperatur oder höherer Temperatur vorhanden sein würde, da bekanntlich hochverdünnte Proteinlösungen, in denen durch die Verdünnung auch der kolloidale Schutzfaktor verringert wird, äußerst instabil sind.

Überraschenderweise zeigte sich jedoch, daß bei der erfindungsgemäßen Verwendung monoklonale Antikörper außergewöhnlich stabil und lange reaktionsfähig sind.

Monoklonale Antikörper sind hochspezifische Antikörper, die sich dadurch herstellen lassen, daß man ein Tier mit einem Antigen immunisiert, Antikörper produzierende Zellen von diesem Tier erhält und die Antikörper produzierenden Zellen mit Tumor-Zell-Linien ; z. B. Myeloma-Zell-Linien, fusioniert, wobei man Hybridomas erhält, die isoliert werden und die die monoklonalen Antikörper produzieren (Köhler, G. und Milstein, C., Eur.J.Immunol., 6, 511-519 (1976) und Köhler, G. und Milstein, C., Nature, 256, 495 (1975).

Monoklonale Antikörper funktionieren wie Antiseren, obgleich sie nicht Bestandteil eines Serums, sondern aus dem Serum « herausgepickte » Antikörper sind.

Die aus dem Kulturmedium von Hybridoma-Kulturen in vitro oder aus den Ascitesflüssigkeiten von tumortragenden Wirtstieren gewonnenen monoklonalen Antikörper reagieren angetrocknet auf festen Phasen als agglutinierende Antikörper in gleicher Weise wie Antiseren oder Immunglobulin-Fraktionen.

Sie haften auf den erfindungsgemäß zu verwendeten festen Phasen ohne jegliche haftvermittelnden Zusätze in genauso ausgezeichneter Weise wie Antiseren.

Ein für die feste Phase besonders geeignetes Folienmaterial ist eine Polyesterfolie, die im Extrudierverfahren hergestellt und durch biaxiale Streckung und Hitzestabilisierung dimensionsstabil gemacht wurde und auf beiden Seiten mit einer gleichmäßigen Pigmentlackmattierung beschichtet ist.

Eine besonders zweckmäßige Folie besitzt folgende mechanische und physikalische Eigenschaften :

Zugfestigkeit 1 800 kg/cm$^2$, Streckgrenze 980 kg/cm$^2$,

thermische Ausdehnung 0,027 mm/m/°C,

Feuchtigkeitsausdehnung 0,01 mm/m/% RF,

Wärmefestigkeit 150 °C

Eine derart besonders zweckmäßige Folie ist unter der Bezeichnung Zeichenfolie Safir PL Opak-weiß im Handel erhältlich.

Als besonders gut geeignete Mikrotiterplatten erwiesen sich Flachbodenplatten mit zylindrischen Näpfchen aus strahlensterilisiertem Polystyrol, wie sie für die Verwendung für Zellkulturen im Costar (R) Kat.Nr. 3596 beschrieben werden. Diese Platten weisen eine hohe Proteinbindungsfähigkeit auf. Die Näpfchenböden können zylindrisch, U-förmig oder V-förmig ausgebildet sein.

Beispiel 1

Es wurden vergleichende Lagerungsversuche zwischen Blutgruppenidentitätskarten aus einer Polyesterfolie, wie sie vorstehend als besonders zweckmäßige Folie beschrieben wurde, durchgeführt, auf die einerseits Anti-A und Anti-B Antiseren und andererseits Anti-A und Anti-B monoklonale Antikörper aufgetrocknet worden waren.

Bei einer Wechsellagerung zwischen 37 °C und 2-8 °C war bei den mit Antiseren beschichteten Karten bereits nach 3 Wochen Gelbildung zu beobachten, während bei den mit monoklonalen Antikörpern beschichteten Karten nach 14 Wochen keine Gelbildung erfolgt war und die Antikörper völlig intakt bzw. reaktiv waren ohne störende Effekte.

Beispiel 2

Man verfuhr nach Beispiel 1, jedoch mit dem Unterschied, daß man die Karten bei Raumtemperatur lagerte. Bei den mit Antiseren beschichteten Karten war bereits nach 6 Wochen Gelbildung zu beobachten, während bei den mit monoklonalen Antikörpern beschichteten Karten nach 27 Wochen keine Gelbildung erfolgt war und die Antikörper völlig intakt bzw. reaktiv waren ohne störende Effekte.

Außer zur Blutgruppenbestimmung lassen sich mit dem erfindungsgemäßen Testsystem auch andere serologische und mikrobiologische Tests durchführen.

**Patentanspruch**

Diagnostisches Testsystem bestehend aus einer festen Phase aus einem proteinbindungsfähigen Polystyrol oder einer Polyesterfolie, die mindestens auf einer Seite mit einer gleichmäßigen gegenüber bestimmten Reagentien haftfähigen Pigmentlackmattierungsschicht versehen ist, auf die in räumlich voneinander getrennten Kompartimenten oder Feldern Reagentien aufgetrocknet sind, dadurch gekennzeichnet, daß als Reagentien monoklonale Antikörper mit Spezifität gegen bestimmte Antigene, insbesondere Blutgruppenantigene verwendet werden.

**Claim**

A diagnostic-test system consisting of a solid phase of a polystyrene that has the capacity to bind protein or of a sheet of polyester film at least one side of which has a uniform pigment-lacquer dulling layer that has the capacity to adhere to specific reagents, the reagents are dried on said phase in spatially separated compartments or fields characterized in that monoclonal antibodies that are specific to certain antigens, especially blood-type antigens, are employed.

**Revendication**

Système de test diagnostique constitué d'une phase solide faite d'un polystyrène apte à se lier aux protéines ou d'une feuille de polyester, qui est munie sur au moins un côté d'une couche de vernis mat pigmenté adhésif vis-à-vis de certains réactifs et régulière, sur laquelle sont séchés des réactifs dans des compartiments ou zones séparés les uns des autres dans l'espace, caractérisé en ce qu'on utilise comme réactifs des anticorps mono-clonaux ayant une spécificité contre certains antigènes, en particulier les antigènes des groupes sanguins.